Europäisches Patentamt

European Patent Office

Office européen des brevets

(11) Veröffentlichungsnummer: **0 244 561**
**A1**

(12) # EUROPÄISCHE PATENTANMELDUNG

(21) Anmeldenummer: **87101371.0**

(22) Anmeldetag: **02.02.87**

(51) Int. Cl.⁴: **A61B 6/04 , A61B 6/00**

(30) Priorität: **07.05.86 DE 8612495 U**

(43) Veröffentlichungstag der Anmeldung:
**11.11.87 Patentblatt 87/46**

(84) Benannte Vertragsstaaten:
**DE FR**

(71) Anmelder: **Siemens Aktiengesellschaft Berlin
und München
Wittelsbacherplatz 2
D-8000 München 2(DE)**

(72) Erfinder: **Bock, Hans-Christian, Dipl.-Ing. (FH)
Siedlung 9A
D-8525 Uttenreuth(DE)**
Erfinder: **Schäfer, Willi, Dipl.-Ing. (FH)
Genglerstrasse 20
D-8520 Erlangen(DE)**
Erfinder: **Kaul, Karlheinz, Dipl.-Ing. (FH)
Ruhsteinweg 4
D-8525 Uttenreuth(DE)**

(54) **Röntgenuntersuchungstisch.**

(57) Die Erfindung bezieht sich auf einen Röntgenuntersuchungstisch mit einem Sockel (2), an dem ein Bedienkästchen (5) mit Bedienelementen angeordnet ist. Das Bedienkästchen (5) ist in Tischlängsrichtung verschiebbar, so daß der Arzt die Bedienelemente auch bei Untersuchungen im Kopfbereich des Patienten leicht erreichen kann.

FIG 2

EP 0 244 561 A1

## Röntgenuntersuchungstisch

Die Erfindung betrifft einen Röntgenuntersuchungstisch mit einem Sockel, an dem ein Bedienkästchen mit Bedienelementen angeordnet ist.

Es ist bekannt, die Steuerung eines Röntgenuntersuchungsgerätes von einem Röntgenuntersuchungstisch aus in der Weise vorzunehmen, daß die Bedienelemente an einem Bedienkästchen des Untersuchungstisches betätigt werden. Der Arzt muß sich nun für manche Untersuchungen, z.B. am Kopf-oder Fußende des Patienten, relativ weit vom Bedienkästchen entfernen. Die Bedienung ist demgemäß für solche Untersuchungen erschwert, denn das Bedienkästchen wird an einem Ende des Untersuchungstisches angeordnet, so daß es das Einführen einer Röntgenfilmkassette unter den Untersuchungstisch nicht stört.

Der Erfindung liegt die Aufgabe zugrunde, einen Röntgenuntersuchungstisch der eingangs genannten Art so auszubilden, daß die Bedienelemente bei allen praktisch vorkommenden Untersuchungen leicht erreichbar sind.

Diese Aufgabe ist erfindungsgemäß dadurch gelöst, daß das Bedienkästchen mit dem Sockel in Tischlängsrichtung verschiebbar verbunden ist. Bei dem erfindungsgemäßen Röntgenuntersuchungstisch kann das Bedienkästchen bei Anordnung an einem Ende des Untersuchungstisches in dem Fall, in dem der Arzt am anderen Ende arbeitet, in Richtung auf die Arbeitsposition des Arztes hin verschoben werden, so daß seine Bedienelemente immer leicht erreichbar sind.

Die Erfindung ist nachfolgend anhand eines in der Zeichnung dargestellten Ausführungsbeispieles näher erläutert. Es zeigen:

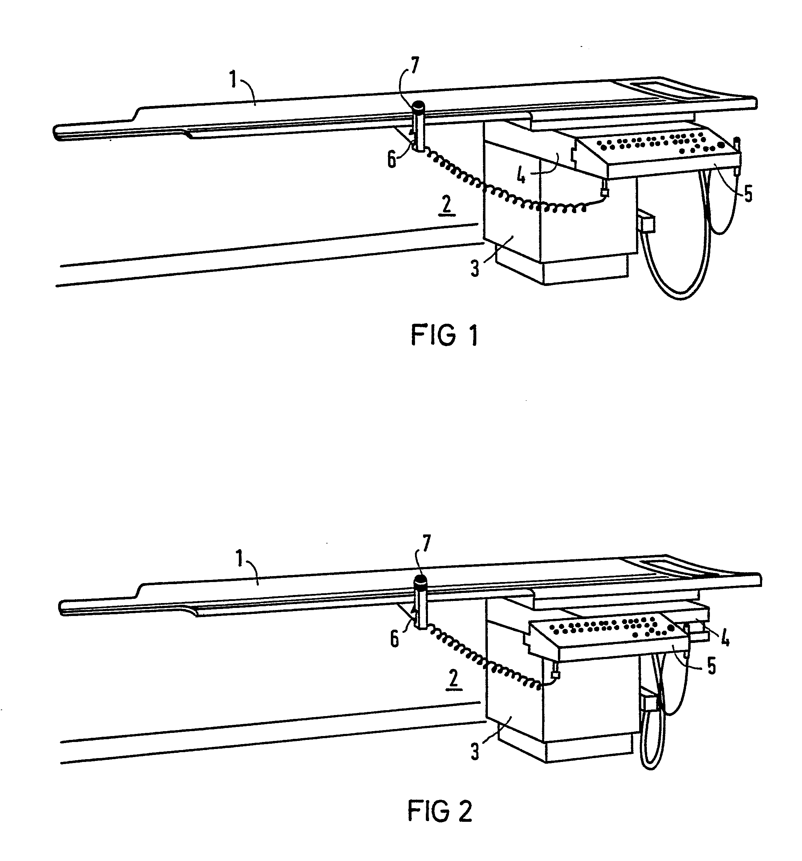

Fig. 1 und 2 einen Röntgenuntersuchungstisch nach der Erfindung mit zwei verschiedenen Stellungen des ihm zugeordneten Bedienkästchens.

In den Figuren 1 und 2 ist eine Tischplatte 1 dargestellt, die frei tragend auf einem höhenverstellbaren Sockel 2 gelagert ist. Der Sockel 2 besteht aus einem Teleskopteil 3, das aus ineinandergeschobenen, kastenförmigen Einzelteilen besteht und aus einem Oberteil 4, mit dem die Tischplatte 1 abnehmbar verbunden ist. Am Oberteil 4 ist ein Bedienkästchen 5 angeordnet. Das Bedienkästchen 5 ist in Tischlängsrichtung zum freien Ende der Tischplatte 1 hin verstellbar. Es weist demgemäß die beiden in den Figuren 1 und 2 dargestellten, gerasteten Arbeitsstellungen auf.

Die Figur 1 zeigt das Bedienkästchen 5 in seiner Normallage. Arbeitet der Arzt im Kopfbereich des Patienten, so ist der Weg zum Bedienkästchen 5 in der Normallage relativ weit. Um dem Arzt die Bedienung zu erleichtern, kann er das Bedienkästchen 5 in die in der Figur 2 gezeigte Lage zum Kopfende der Tischplatte 1 hin verschieben, so daß es leichter erreichbar ist. Flexible Leitungen zu den Bedienelementen ermöglichen diese Verschiebung.

Die Tischplatte 1 weist ferner einen Halter 6 für ein Steuerelement 7 auf, das am Bedienkästchen 5 gemäß Figur 1 anschließbar ist. Dadurch ist ebenfalls eine einfache Steuerung bestimmter Vorgänge von der Tischplatte 1 aus möglich.

### Ansprüche

1. Röntgenuntersuchungstisch mit einem Sockel (2), an dem ein Bedienkästchen (5) mit Bedienelementen angeordnet ist, **dadurch gekennzeichnet,** daß das Bedienkästchen (5) mit dem Sockel (2) in Tischlängsrichtung verschiebbar verbunden ist.

2. Röntgenuntersuchungstisch nach Anspruch 1, **dadurch gekennzeichnet,** daß an der Tischplatte (1) ein Halter (6) für weitere Bedienelemente (7) vorgesehen ist.

FIG 1

FIG 2

## Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 298 801 (C.J. HEITMAN et al.) <br> * Spalte 1, Zeilen 5-11; Spalte 4, Zeilen 20-50; Spalte 5, Zeilen 23-60; Figuren 1,2 * | 1 | A 61 B 6/04 <br> A 61 B 6/00 |
| Y | DE-A-2 260 140 (AKTIEBOLAGET STILLE-WERNER) <br> * Seite 3, Zeilen 1-10; Seite 7, Zeilen 9-14; Figur 1 * | 1 | |
| A | FR-A-2 255 038 (SIEMENS AG) <br> * Seite 1, Zeilen 1-8; Seite 2, Zeile 29 - Seite 3, Zeile 10; Figuren 1,2 * | 1,2 | |
| A | FR-A-1 400 356 (SIEMENS-REINIGER-WERKE) <br> * Seite 1, linke Spalte, Zeilen 1-7; Seite 2, linke Spalte, Zeilen 4-33; Figuren 1,2 * | 1 | RECHERCHIERTE SACHGEBIETE (Int. Cl.4) <br><br> A 61 B <br> A 61 G |
| A | DE-A-3 226 374 (SIEMENS AG) <br> * Zusammenfassung; Seite 3, Zeilen 8-13; Seite 5, Zeilen 5-18; Figuren 1,2 * | 2 | |

Der vorliegende Recherchenbericht wurde für alle Patentansprüche erstellt.

| Recherchenort | Abschlußdatum der Recherche | Prüfer |
|---|---|---|
| DEN HAAG | 12-08-1987 | FERRIGNO, A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82